(19) 

Europäisches
Patentamt
European
Patent Office
Office européen
des brevets

(11) **EP 2 041 306 B1**

(12) **EUROPEAN PATENT SPECIFICATION**

(45) Date of publication and mention
of the grant of the patent:
**16.06.2010 Bulletin 2010/24**

(21) Application number: **07765688.2**

(22) Date of filing: **28.06.2007**

(51) Int Cl.:
*C12Q 1/68* [(2006.01)]     *G01N 33/50* [(2006.01)]

(86) International application number:
**PCT/EP2007/056458**

(87) International publication number:
**WO 2008/000785 (03.01.2008 Gazette 2008/01)**

(54) **A SCREENING METHOD FOR IDENTIFYING NEW DRUGS**

SCREENING-VERFAHREN ZUR IDENTIFIZIERUNG NEUER ARZNEISTOFFE

PROCÉDÉ DE CRIBLAGE DESTINÉ À IDENTIFIER DE NOUVEAUX MÉDICAMENTS

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **28.06.2006 EP 06116233**

(43) Date of publication of application:
**01.04.2009 Bulletin 2009/14**

(73) Proprietors:
• **Institució Catalana de Recerca i Estudis
Avançats
(ICREA)
08010 Barcelona (ES)**
• **Fundació Privada Parc Científic de Barcelona
08028 Barcelona (ES)**
• **Fundació Privada Institut de Recerca Biomèdica
08028 Barcelona (ES)**

(72) Inventors:
• **RIBAS DE POUPLANA, Lluís
E-08028 Barcelona (ES)**
• **BORI SANZ, Teresa
E-08015 Barcelona (ES)**

(74) Representative: **ZBM Patents
Zea, Barlocci & Markvardsen
Plaza Catalunya, 1
08002 Barcelona (ES)**

(56) References cited:
**EP-A- 0 785 258     EP-A2- 1 300 468
WO-A-99/18239     WO-A2-02/04611
US-B1- 6 475 726**

**Description**

The present invention relates to a new screening method which permits the identification of new drugs. Particularly, the present invention refers to a screening method for the selection of aminoacyl-tRNA synthetase (ARS) inhibitor compounds which can be useful as antibacterial and antifungal agents, among others.

BACKGROUND ART

In modern drug discovery programs chemical libraries are used in combination with robotic systems to rapidly evaluate the effect of large numbers of compounds on a given reaction. This approach has two major drawbacks. First, a biochemical assay that can be easily monitored often needs to be developed in order to identify candidate compounds. This process is costly and insensitive due to potential negative effects of the selected drugs. Secondly, this approach ignores bioavailability and toxicity parameters. Most of the compounds initially selected are later discarded due to solubility, bioavailability, or toxicity problems.

Aminoacyl-tRNA synthetases (hereinafter so-called "ARSs") represent ideal targets for drug development because they are essential enzymes of universal distribution, whose ancestral nature allows for the selection of specific inhibitors. In addition, they are soluble, stable, easy to express and purify in large amounts, and are straightforward to assay by one or more methods. X-ray structures are available for examples of all synthetases, and much is known about the mechanism of the aminoacylation reaction (cf., Weygand-Durasevic I. et al., "Yeast seryl-tRNA synthetase expressed in Escherichia coli recognizes bacterial serine-specific tRNAs in vivo", Eur. J. Biochem., 1993, vol. 214, pp. 869-877).

ARSs catalyze the ligation of specific amino acids to cognate tRNAs. This reaction takes place within a single active site domain and typically proceeds in two steps. First, the amino acid is activated with ATP to form aminoacyladenylate with release of pyrophosphate. Next, the amino acid is transferred to the 3'-end of the tRNA to generate aminoacyl-tRNA and AMP. This two-step reaction establishes the genetic code by linking specific nucleotide triplets triplets (tRNA anticodons) with specific amino acids. Each amino acid is recognized by its own specific ARS, which is universally distributed. The aminoacyl-tRNA synthetases are evenly divided into two classes of approximately 10 enzymes each. All enzymes within a class appear to have evolved from a single-domain ATP binding protein. Insertions into and variations on this domain established a framework for binding the tRNA acceptor stem. Over the course of evolution additional domains were added to this core structure.

The recognition of tRNAs by aminoacyl-tRNA synthetases depends mostly on molecular interactions with the acceptor system and the anticodon loop of the tRNA (cf. Rich, A. "RNA structure and the roots of protein synthesis", Cold Spring Harb. Symp. Quant. Biol., 2001, vol. 66, pp. 1-16). The active site domains of the enzymes bind to the acceptor arm of the tRNA molecule, where the amino acid is attached. The 'discriminator' base (the unpaired base that precedes the universal CCA sequence), and the first three base pairs of the acceptor stem harbor most identity elements recognized by ARS active sites.

US 6 475 726 describes a method for identifying one or more compounds that are candidates for binding to a target cell component in a pathogen and inhibiting infection of a mammal by the pathogen comprising: a) constructing a pathogen comprising a regulable gene encoding a biomolecule which binds to the target cell component; b) infecting one or more test animals with the constructed pathogen, and one or more control animals with the constructed pathogen or with a control pathogen; c) regulating expression of the regulable gene to produce the biomolecule in the test animals; d) monitoring the test animals and the control animals for signs of infection, wherein observing fewer or less severe signs of infection in the test animals than in the control animals indicates that the biomolecule is a biomolecular inhibitor of infection by the pathogen; and e) identifying one or more compounds that compete with the biomolecular inhibitor of infection for binding to the target cell component in a competitive binding assay; whereby, if a compound competes with the biomolecular inhibitor of infection for binding to the target cell component, then the compound is a candidate for binding to the target cell component in the pathogen and inhibiting infection of a mammal by the pathogen.

WO 02/04611 describes human aminoacyl tRNA synthetases (ATRS) and polynucleotides which identify and encode said synthetases.

EP 785 258 and EP 1 300 468 describe isolated nucleic acid molecules encoding tRNA synthetase, particularly from *Staphylococcus aureus* tRNA synthetase, including mRNAs, cDNAs, genomic DNAs.

Among the translation-directed commercial antibiotics one is targeted to a tRNA synthetase. Pseudomonic acid (mupirocin) is an inhibitor of isoleucyl-tRNA synthetases (IIeRS) from Gram-positive infectious pathogens. Pseudomonic acid has an approximate 8000-fold selectivity for pathogen vs. mammalian IIeRS, but the drug's lack of systemic bioavailability limits its use to topical applications.

Although other known natural product inhibitors directed against synthetases exist (e.g., borrelidin, furanomycin, granaticin, etc.), none of these has been developed into commercial antibiotics due to lack of inhibitory activity, poor specificity, or poor bioavailability. Thus, a more efficient method for selecting ARS inhibitors is required to screen large chemical libraries and identify promising drug candidates.

## SUMMARY OF THE INVENTION

The aim of the present application is to provide a screening method for the selection of ARS inhibitors.

It is provided a positive screening method which implies that the desired effect of a potential lead compound is the rescue and/or stimulation of growth of mammalian cells, and not the inhibition of any given reaction or the arrest in growth of a cellular culture. Thus, in the positive selection that here it is proposed, the growth of mammalian cells (and in particular of human cells) would be rescued by those molecules capable of inhibiting the toxic action of a target ARS. This effect could be monitored simply by measuring culture density, a fast and cheap procedure.

Thus, an aspect of the present invention is the provision of a screening method for identifying a candidate to drug, said method comprising the steps of: a) obtaining an expression vector which comprises a gene sequence codifying a naturally occurring pathogenic non-discriminating tRNA synthetase; b) transforming isolated mammalian cells with the expression vector; c) growing the recombinant cells resulting from (b) in a nutrient medium under conditions which allow the expression of the pathogenic tRNA synthetase, resulting the expression of the pathogenic tRNA synthetase into cell death or decrease in the rate of cell division; d) providing a substance to be tested; and e) analyzing the resulting cell growth, wherein if there is an increase in cell growth, then the substance selectively inhibits the activity of the pathogenic tRNA synthetase and does not affect to its cellular ortholog, resulting that said substance is a candidate to drug.

Preferably, the isolated mammalian cells are isolated human cells.

The screening method of the present invention is based on the strategy of if a non-specific ARS is toxic to cells, and this toxicity can be achieved without the manipulation of the active site, then it should be possible to engineer the ARS of a human pathogen to mischarge human tRNAs, thus killing, or affecting the growth rate of, the mammalian cells that happened to express this protein. If the candidate molecule to drug happens to be a compound that binds to the active site of the enzyme then they would be equally active in the wild-type ARS from the pathogen of interest, because the catalytic cavity of the toxic ARS has not been manipulated in this process.

Advantageously, the molecules identified as drug candidates following the screening method of the present invention are characterized as been small molecules selected due to their ability to revert the toxic effect of the non-specific ARS, but also as been able to, simultaneously, cross the cellular membrane, inhibit the pathogenic synthetase, not inhibit its human orthologs, and not affect other aspects of the cell metabolism. Therefore, said drug candidates kills specifically the pathogen and are not toxic for the host cell.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and variations of the word, such as "comprising", are not intended to exclude other technical features, additives, components, or steps. Additional objects, advantages and features of the invention will become apparent to those skilled in the art upon examination of the description or may be learned by practice of the invention. The following examples and drawings are provided by way of illustration, and are not intended to be limiting of the present invention.

## BRIEF DESCRIPTION OF THE DRAWINGS

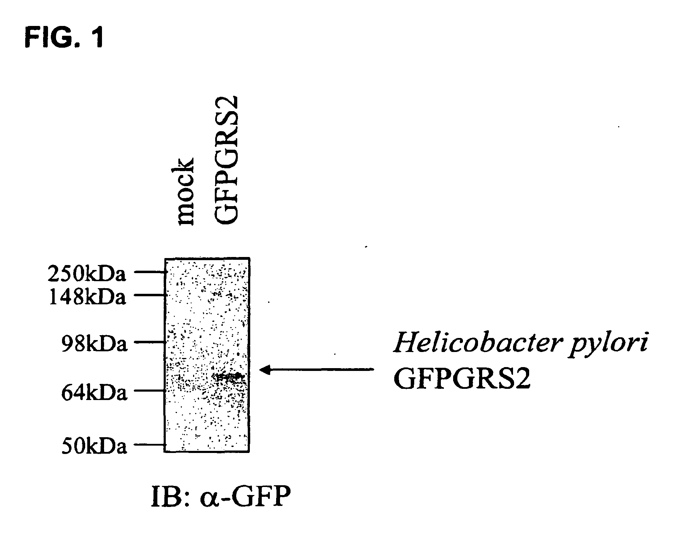

FIG. 1 represents the expression of *H. pylori* GFPGRS2 in HeLa cells. Mock or transiently transfected with *H.pylori* GFPGRS2 whole cell lysates were subjected to SDS/PAGE under reducing conditions. GFPGRS2 was detected by immunoblotting using a polyclonal anti-GFP antibody.

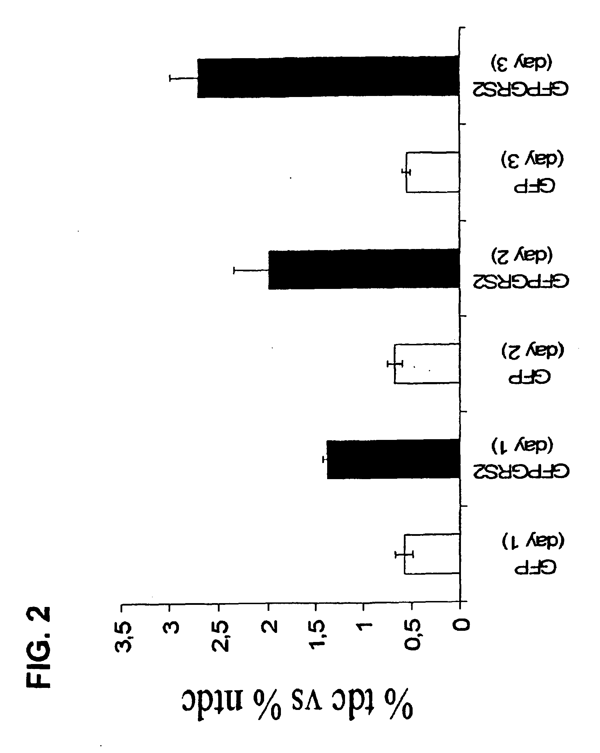

FIG. 2 shows that the expression of *H. pylori* GFPGRS2 leads to an increase of cell death. GFP (in grey) or *H.pylori* GFPGRS2 (in black) were transiently transfected in HeLa cells and 16 hr later, fresh media was added. Cell death was examined by PI staining one, two or three days after having added fresh media. Ratio of the percentage of dead transfected cells (abbreviated as "dtc") versus the percentage of dead non-transfected cells (abbreviated as "dntc") is displayed in the y axis. Triplicate samples were counted for each condition and standard deviation are shown.

## DETAILED DESCRIPTION OF PARTICULAR EMBODIMENTS

As used herein, the term "non-discriminating ARN-t synthetase" (abbreviated as "ND ARN-t synthetase") refers to an aminoacyl-tRNA synthetase whose biological function is to aminoacylate more than one type of tRNA with the same amino acid. For instance, most bacteria contain a GluRS enzyme that is capable of aminoacylating tRNA$^{Gln}$ with glutamate instead of glutamine, its cognate amino acid. This reaction is not toxic to the cells that harbor this enzyme because the transient form of tRNA$^{Gln}$ aminoacylated with glutamate is rapidly modified and the amino acid glutamate is transformed to glutamine. As used in the present invention, the terms "non-specific" or "non-canonical" have the same meaning than the term "non-discriminating".

The term "naturally occurring pathogenic" is to be understood as that the non-discriminating tRNA synthetase according to the present invention is obtained from organisms that are pathogenic for mammals and which have not been genetically engineered.

In one embodiment of the first aspect of the invention the expression vector obtained in step (a) also comprises a gene sequence codifying for a tRNA substrate of the naturally occurring pathogenic non-discriminating tRNA synthetase.

In another embodiment of the first aspect of the invention the mammalian cells are transformed in step (b) using a second expression vector comprising a gene sequence codifying for a tRNA substrate of the naturally occurring pathogenic non-discriminating tRNA synthetase.

The inventors of the present invention have found that the simultaneous expression of the genes coding for the pathogenic non-discriminating tRNA synthetase and its tRNA substrate can increase the ability of the said non-discriminating tRNA synthetase to induce toxicity in the cells that express both genes.

Gene sequences codifying for a tRNA substrate of naturally occurring pathogenic non-discrimating tRNA synthetases are available from public databases (for instance, Helicobacter pylori complete genome sequences from three different isolates can be found under Genebank references NC_000915.1, NC_008086.1, and NC_000921.1).

In one embodiment of the present invention, the naturally occurring pathogenic non-discriminating t-RNA synthetase is selected from the group consisting of Glu-tRNA synthetase and Asp-tRNA synthetase.

Among the aminoacyl-tRNA synthetases, the glutamyl- and glutaminyl-tRNA synthetases (GluRS and GlnRS, respectively) and the aspartyl- and asparaginyl-tRNA synthetase (AspRS and AsnRS respectively) are respectively related in sequence and evolutionarily (cf. Brown, J. R. et al., "Gene descent, duplication, and horizontal transfer in the evolution of glutamyl- and glutaminyl-tRNA synthetases", J. Mol. Evol. 1998, vol. 49, p. 485-495; Hong, K. W.,et al., "Retracing the evolution of amino acid specificity in glutaminyl-tRNA synthetase", FEBS Lett. 1999,. vol. 434, p. 149-154).

In eukarya and some bacteria, GlnRS and GluRS (GluRS-D) each catalyze a highly specific tRNA aminoacylation reaction. GluRS-D does not misacylate $tRNA^{Gln}$ with Glu, and GlnRS does not misacylate $tRNA^{Glu}$ with Gln.

In contrast, the archaea and most bacteria do not encode a functional GlnRS, and Gln-$tRNA^{Gln}$ is biosynthesized indirectly (cf., Wilcox, M. & Nirenberg, "Transfer RNA as a cofactor coupling amino acid synthesis with that of protein", 1968, Proc. Natl. Acad. Sci. USA, vol. 61, p. 229-236.)

First, $tRNA^{Gln}$ is misacylated by a nondiscriminating GluRS (GluRS-ND), to form Glu-$tRNA^{Gln}$ (Equation 1). [GluRS-ND still catalyzes its cognate reaction, to generate Glu-$tRNA^{Glu}$]. Next, the misacylated Glu-$tRNA^{Gln}$ intermediate is transamidatively modified by the glutamine-dependent Glu-$tRNA^{Gln}$ amidotransferase (Glu-Adt) (Equation 2):

$$\text{Glu} + tRNA^{Gln} + \text{ATP} + \text{GluRS-ND} \rightarrow \text{Glu-}tRNA^{Gln} + \text{AMP} + \text{PPi} \quad \text{(Eq. 1)}$$

$$\text{Gln} + \text{Glu-}tRNA^{Gln} + \text{ATP} + \text{Glu-Adt} \rightarrow \text{Gln-}tRNA^{Gln} + \text{Glu} + \text{ADP} \quad \text{(Eq. 2)}$$

There is an analogous situation for the AspRS and AsnRS:

$$\text{Asp} + tRNA^{Asn} + \text{ATP} + \text{AspRS-ND} \rightarrow \text{Asp-}tRNA^{Asn} + \text{AMP} + \text{PPi} \quad \text{(Eq. 3)}$$

$$\text{Asn} + \text{Asp-}tRNA^{Asn} + \text{ATP} + \text{Asp-Adt} \rightarrow \text{Asn-}tRNA^{Asn} + \text{Asp} + \text{ADP} \quad \text{(Eq. 4)}$$

In this way, the fidelity of the genetic code is accurately maintained, despite the absence of a cognate GlnRS or AsnRS.

However, the introduction of a NDGluRS into a cell that is not capable of catalyzing this modification step is toxic to the cell, because it results in the accumulation of $tRNA^{Gln}$ aminoacylated with glutamate, eventually causing massive mutagenesis in the proteins being synthesized by the organism. This effect has been proven by expressing an NDGluRS in *Escherichia coli*, an organism that lacks the ability to transform glutamate in $tRNA^{Gln}$ to glutamine.

In the present invention "an expression vector" refers to a carrier molecule to which a desired segment of DNA (e.g. heterologous nucleic acid) is inserted. The vector serves to incorporate foreign DNA into host cells. More particularly, an "expression vector" is a DNA vector containing a DNA sequence which is operably linked to a suitable control sequence capable of effecting the expression of the DNA in a suitable host. Such control sequences include a promoter to effect transcription, an optional operator sequence to control such transcription, a sequence encoding suitable mRNA ribosome binding sites, and sequences which control the termination of transcription and translation. The vector may be a plasmid, a phage particle, or simply a potential genomic insert. Once transformed into a suitable host, the vector may replicate and function independently of the host genome, or may in some instances, integrate into the genome itself, generating stable cell lines that express said gene constitutively or after the treatment of the cells with an inducer of the expression of the gene. The terms "plasmid" and "vector" are sometimes used interchangeably herein, because the plasmid is the most commonly used form of vector at present. However, the invention is intended to include such other forms of vector that serve an equivalent function and are or become known in the art.

Expression vectors typically further contain other functionally important nucleic acid sequences, such as expression cassettes encoding antibiotic resistance proteins, multiple cloning sites, replication sequences, and the like.

In one embodiment of the present invention, the expression vector is selected from the group consisting of a viral or non-viral plasmid, cosmid, phagemid, shuttle vector, yak, and the like. Preferably the expression vector is an adenovirus.

In another embodiment of the present invention, the vector further comprises a tetracycline-dependent regulation system for the expression of the gene.

In still another embodiment of the invention the vector comprises a selection marker. Preferably the selection marker is hygromicine.

In still yet another embodiment, the pathogen is selected from the group consisting of organisms that utilize non-specific ARS for the translation of their genetic code like, for instance, *Streptococcus pneumoniae.*

As used herein, the terms "transformation" and "transfection" refer to any of the variety of art-recognized techniques for introducing foreign nucleic acid (e. g. DNA) into either a prokaryotic or eukaryotic host cell (including isolated human cells). Suitable means for introducing (transducing) expression vectors containing nucleic acid into host cells to produce transduced recombinant cells, or to generate stable cell lines containing the gene integrated in the nuclear DNA of the cells are well-known in the art. Suitable methods for transforming or transfecting host cells can be found in Molecular Cloning: A Laboratory Manual,3rd edition, edited by J. Sambrook and D. W. Russell (Cold Spring Harbor Laboratory Press, Cold Spring Harbor, NY, 2000), and other laboratory manuals.

Methods for the growth and preservation of bacterial strains are disclosed in Molecular Cloning: A Laboratory Manual,3rd edition, edited by J. Sambrook and D. W. Russell (Cold Spring Harbor Laboratory Press, 2000).

Controlling the expression of genes in human cells and repressing the existence of basal expression can be challenging as it is well-known for the skilled person in the art (cf., Rai et al., "Expression systems for production of heterologous proteins", Current Science, 2001, vol. 80, pp. 1121-11). The inventors have been taken advantage of two recent developments in the field of protein expression of human cells to vector our tester strains. First, it has been used an adenovirus-based gene expression vector based on the tetracycline-regulated Tet-ON- and the progesterone antagonist RU 486-regulated gene expression systems. This vector can function in a number of cell types and the regulation of protein expression was shown to be tightly controlled (cf., Edholm, D. et al., "Adenovirus vector designed for expression of toxic proteins", J. Virology, 2001, vol. 75, pp. 9579-9584).

Alternatively, the vector of tester strains could be based on the Cre/loxP recombination system for the activation of gene transcripts. Cre is a 38 kDa recombinase protein from bacteriophage P1 which mediates intramolecular (excisive or inversional) and intermolecular (integrative) site specific recombination between loxP sites. The Cre's DNA excising capability can be used to turn on a foreign gene by cutting out an intervening stop sequence between the promoter and the coding region of the gene. Thus, the genes coding for the toxic synthetases could be introduced in human cells in a vector whose transcription initiation site is blocked by a stop signal. The recombination, i.e. excision of the stop signal, occurs only when the expression of Cre is activated (cf., Sauer, B et al., "Cre/lox: one more step in the taming of the genome", Endocrine, 2002, Vol. 19, pp. 221-228).

Once the tester strains are developed the inventors have designed a simple growth-monitoring test in 96-well plates using an automatic plate reader. They have already managed to implement a similar procedure for the analysis of the toxic enzymatic effect in *E. coli.* Once this test is operational it is started the screening of small molecule libraries to look for potential new inhibitors of target synthetases.

The terms "test substance" and "substance" are used interchangeably and refer to a compound, a mixture of compounds (i. e., at least two compounds), or a natural product sample containing one or more compounds. Thus, the analysis of the cell growth of a test substance may encompass the inhibitory activity of more than one growth inhibitor, such that combinations of selective and non-selective inhibitors of the targeted gene product have an observable related to the growth of the eukaryotic cells that contain the toxic non-discriminating ARS.

Short of testing the effect of small molecules in whole tissues or individuals, testing them in human cell cultures would provide the screens with the highest possible discriminatory power, because the selection based on cell growth identifies compounds or combinations of compounds on a multi-factorial basis. Initial selections identify inhibitors capable of blocking the activity of the synthetase and of traversing cellular membranes, while the second screen further refine the search for molecules that did not affect human cells metabolism.

Human cells expressing the non-discriminating aminoacyl-tRNA synthetases are used for the discovery of inhibitors of those enzymes. The cells that contain the genes coding for the non-discriminating aminoacyl-tRNA synthetases synthesize these proteins when the expression of the same genes is induced, and this causes cell death due to the biochemical reaction catalyzed by the non-discriminating aminoacyl-tRNA synthetases.

The cell death caused by the non-discriminating aminoacyl-tRNA synthetases can be monitored by a variety of commercial or standard methods (neutral red uptake, WST1, LDH levels, ATP levels, and others), using spectrophotometers or any other device designed for the purpose of monitoring cell death.

Compounds to be tested for their ability to eliminate the toxic effect caused by the non-discriminating aminoacyl-tRNA synthetases are added to the cells before, during, or after the induction of the expression of the genes coding for the non-discriminating aminoacyl-tRNA synthetases. After induction of the genes, the death of cells in each cell culture is monitored in the presence or absence of each of the compounds that are being tested.

The compounds that cause a reduction in the rate of cell death of the culture with respect to the rate of cell death of the same culture in the absence of the compound are considered potential inhibitors of the non-discriminating aminoacyl-tRNA synthetases that cause cell death.

Thus, in one embodiment of the present invention the naturally occurring pathogenic non-discriminating tRNA synthetase comes from a bacterium and the substance is being tested to determine whether it is an antibacterial agent that acts by selectively inhibiting the function of the non-discriminating t-RNA synthetase of bacterial origin expressed into the recombinant human cell.

In another embodiment of the present invention the naturally occurring pathogenic non-discriminating tRNA synthetase comes from a fungus and the substance is being tested to determine whether it is an antifungal agent that acts by selectively inhibiting the function of the non-discriminating t-RNA synthetase of bacterial origin expressed into the recombinant human cell.

In another embodiment of the present invention the naturally occurring pathogenic non-discriminating tRNA synthetase comes from a protozoan and the substance is being tested to determine whether it is an anti-parasite agent that acts by selectively inhibiting the function of the non-discriminating t-RNA synthetase of protozoan origin expressed into the recombinant human cell.

In yet another embodiment of the present invention the naturally occurring pathogenic non-discriminating tRNA synthetase comes from a metazoan and the substance is being tested to determine whether it is an inhibitory agent that acts by selectively inhibiting the function of the non-discriminating t-RNA synthetase of metazoan origin expressed into the recombinant human cell.

EXAMPLE: *Helicobacter pylori* glutamyl-tRNA synthetase expression in HeLa cells

Introduction

The pathogenic bacterium *Helicobacter pylori* utilizes two essential glutamyl-tRNA synthetases (GluRS1 and GluRS2). GluRS1 is a canonical discriminating GluRS and GluRS2 is non-canonical as it is only essential for the production of misacylated Glu-tRNA$^{Gln}$.

To investigate whether expression *H. pylori* non-canonical GRS2 has a toxic effect in a mammalian system and leads to cell death, *H. pylori* GFPGRS2 was expressed in HeLa cells, a human cell line and its putative toxic effect was examined.

Obtaining the expression vector

Plasmids vectors GRS2#1 (SEQ ID NO: 1)
5'-GTCACCACCATGCTTCGTTTTGCGCCTTCGCCTACAG

and GRS2#2 (SEQ ID NO: 2)
5'-GACTCAATGGTGATGGTGATGATGTGCTTTGAGCCTTAAAACTT

were used to amplify GRS2 from genomic DNA from Helicobacter pylori (ATCC 700392D) and to add a kozak consensus ribosome binding side sequence and a His-tag epitope in its C-terminal. The green fluorescent protein (hereinafter so-called "GFP") was fused to the NH$_2$ terminus of GRS2 to ease its detection by flow cytometry and immunoblotting, using standard overlapping PCR techniques.

GFP was amplified using GFP-GRS2#1 (SEQ ID NO: 3)

5-ATCTCCACCATGGTGAGCAAGGGCGAGGAG

and GFP-GRS2#2 (SEQ ID NO: 4)

5'-CTGTAGGCGAAGGCGCAAAACGAAGCTTGTACAGCTCGTCCATGC CGA,

and it was joined to GRS2 using GFP-GRS2#3 (SEQ ID NO: 5)

5'-TCGGCATGGACGAGCTGTACAAGCTTCGTTTTGCGCCTTCGCCTA CAG

and GFP-GRS2#4 (SEQ ID NO: 6)
5'-GATATTCAATGGTGATGGTGATGATGTGCTTTGAGCCTTAAAACTT

The two segments were subsequently mixed together with primers GFP-GRS2#1 and GFP-GRS2#4, and a secondary overlap PCR was performed. Amplified product was cloned into PCR2.1-TOPO/TA (Invitrogen). GFPGRS2/PCR2.1-TOPO/TA was digested with NotI and inserted to similarly cut mammalian expression vector, pCMV (BD Biosciences Clontech).
GFPGRS2/ PCR2.1-TOPO/TA was digested with XbaI and SpeI and inserted to a XbaI cut mammalian tetra-cycline-induced expression vector pTRE2 (BD Biosciences Clontech). pTRE2 was used to express GRS2 in Tet-On HeLa cell line.
pTRE2 is a response plasmid which contains a tetracyctine-responsive $P_{hCMV-1}$ promoter (commercially obtained). This promoter contains the Tet Response Element (TRE), which consists on seven copies of the 42-pb tet operator sequence (*tetO*). The TRE is just upstream of the minimal CMV promoter which lacks the enhancer that is part of the complete CMV promoter.
For double-stable Hela Tet-On transfectants, hygromycine resistance were digested with PvuII and XmnI (which generate blunt ends) from pcDNA3.1/hygromycine vector (Invitrogen) and subcloned into a blunt ended Zral-digested-GFPGRS2/pTRE2.
The integrity and authenticity of all the vectors were confirmed by determination of their nucleotide sequence by standard sequencing techniques.

Obtaining cells

Tet-On HeLa cells are human cervical carcinoma-derived cells that express the reverser tetracycline-controlled transactivator (rtTA) and were obtained from BD Biosciences Clontech.
HeLa cells and HeLa Tet-On were grown in DMEM medium supplemented with 100 U/ml of penicillin, 100 $\mu$g/mL streptomycin and 10% heat-inactivated fetal bovine serum (from Gibco) under 5% $CO_2$/95% air in humidified incubator. HeLa Tet-On cells were maintained in the presence 100 $\mu$g/mL G418.
Cells were kept exponential phase of growth. Adherent cells were deattached by incubating with trypsine-EDTA solution for 5 minutes at 37 ˚C before washing.

Transfection of cells

For transient transfections, 20 $\mu$g of each DNA was added to 500 $\mu$l water containing 252 mM $CaCl_2$. Then, 500 $\mu$l of 2xHepes-buffered-saline buffer (280 mM NaCl, 10 mM KCl, 1.5 mM $Na_2HPO_4$, 50 mM HEPES, 12 mM dextrose, pH 7.1) was added to the DNA mixture drop by drop. 16 hours after adding this transfection mixture to the cells, the medium containing DNA was removed and new medium was added. After 24 hours, transfected cells were used for experimentation.
For double-stable transfectants, Tet-On HeLa cells were transfected with 20 $\mu$g of DNA. 48 hours after transfection, cells were split in 24-well plates and 500 μg/mL of hygromycin for selection was added. Approximately, 15 days later, individual clones of cells were selected and put in 96-well plates for expansion. Clones were selected by checking its expression GFP by flow cytometry.

Selection of clones

Immuno-blotting

Whole protein extracts in Laemmli blue sample buffer were loaded and separated by SDS-PAGE on 10% gels. Gels were transferred to PVDF membranes and blocked with TBS-T (0.5 M Tris, 1.5 M NaCl), 0.1% (v/v) Tween-20, pH 7.4) containing 10% (w/v) milk for at least 1 hour. Subsequently blots were incubated with purified anti-green fluorescent protein rabbit polyclonal antibody (Immunokontact) at 1:5000 in 10% BSA/TBS-T blocking solution. Blots were washed twice immediately following incubation with primary antibody and then another two times at 15 minutes intervals. Finally blots were incubated with secondary antibody (anti-rabbit IgG, horsedish peroxidase linked whole antibody which was supplied by Amersham) at 1:10000 in TBS-T for 1 hour before washing as before and development using an enhanced chemiluminescence (ECL) detection system (Amersham).

Flow cytometry studies

10 μg/ml propidium iodide (PI) was used for determination of cell viability in transiently GFP fusion proteins transfected HeLa cells. PI-stained cells were analysed immediately using a Coulter Epics XL (Beckman Coulter) and analysed using System II software.
From the results obtained using these techniques it was concluded that HeLa cells (from human cervical carcinoma) were transiently transfected with empty plasmid (mock) or plasmid encoding GFP-GRS2/pCMV.
Expression of GFP fusion proteins was detected by immunoblotting using anti-GFP polyclonal antibody, as shown in FIG. 1. A band of approximately 78 kDa was detected in GFP-GRS2 HeLa cells whole cell lysates.

Determination of cell death.

In order to assess whether expression H. pylori non-discriminating GRS2 has a toxic effect in a mammalian system and leads to cell death, H. pylori GFPGRS2 was expressed in HeLa cells, a human cell line and its putative toxic effect was examined.
As shown in FIG. 2, *H. pylori* GFPGRS2 expression in HeLa cells led to an increase of cell death, measured by PI staining.
*H. pylori* GFPGRS2 expression had a deleterious effect of cell survival compared to GFP expression in HeLa cells and this effect is continued up to 3 days post-transfection.
These results demonstrate that expression of *H. pylori* GFPGRS2 in a mammalian system leads to an increase in cell death.
This increase in cell death sensibility might be due to an increase of the incorporation of glutamic acid instead of glutamine that might cause an increased level of misfolded proteins in *H. pylori* GFPGRS2 expressing HeLa cells, leading to an increase of cell death.
Since *H. pylori* GFPGRS2 constitutive expression in HeLa cells leads to an increase in cell death, the inventors have stably expressed *H. pylori* GFPGRS2 in HeLa Tet-On cells, a tetracycline-inducible system.

Determination of a candidate drug

In order to determine if a substance is a candidate drug, this must allow or improve the growth of human cells in the presence of NDGlu tRNA synthetase (i.e., this compound must have an inhibitory activity against said non-discriminating ARS).
It is carried out a biochemical reaction wherein the NDGlu tRNA synthetase catalyzes the incorporation of the corresponding amino acid to the cognate tRNA. This incorporation is monitored using a radioactively labeled amino acid, and measuring the addition of the radioactive label to the tRNA molecule. A similar reaction is catalyzed by the human enzymes homologous to the ARS whose inhibition is sought.
Then, the substance candidate to drug is added to the reaction mixture. The ability of the substance to selectively discriminate between the non-discriminating ARS and its human homologue is estimated by measuring the capacity of the molecule to inhibit the incorporation of the radioactive amino acid to its cognate tRNA$^{Glu}$, and comparing this activity to the ability of the same compound to inhibit the activity of similar human enzymes on their respective cognate tRNAs. A molecule is specific when it can inhibit the enzyme from the pathogenic, but not the similar human enzymes.

Consequently, the molecule is tested for its ability to inhibit the growth of the organism that originally contains the non-discriminating ARS. The molecule that displays selective inhibition of the non-discriminating ARS and the ability to retard or stop the growth of the organism that naturally contains the non-discriminating ARS is considered a potential drug candidate useful to inhibit the growth this organism.

**Claims**

1.  A screening method for identifying a candidate to drug which is a pathogenic amino acyl-tRNA synthetase inhibitor wherein said method comprises the following steps:

    a) obtaining an expression vector which comprises a gene sequence encoding a naturally occurring pathogenic non-discriminating tRNA synthetase;
    b) transforming isolated mammalian cells with the expression vector;
    c) growing the recombinant cells resulting from (b) in a nutrient medium under conditions which allow the expression of the pathogenic tRNA synthetase, which results in cell death;
    d) providing a substance to be tested; and
    e) analyzing the resulting cell growth, wherein if there is an increase in cell growth with respect to the cell growth of the same culture in the absence of the substance to be tested, then the substance selectively inhibits the activity of the pathogenic tRNA synthetase and does not affect to its cellular ortholog, indicating that said substance is a candidate to drug.

2.  The method according to claim 1, wherein the expression vector obtained in step (a) also comprises a gene sequence encoding for a tRNA substrate of the naturally occurring pathogenic non-discriminating tRNA synthetase.

3.  The method according to claim 1, wherein the mammalian cells are transformed in step (b) using a second expression vector comprising a gene sequence encoding for a tRNA substrate of the naturally occurring pathogenic non-discriminating tRNA synthetase.

4.  The method according to any of the preceding claims, wherein the naturally occurring pathogenic non-discriminating t-RNA synthetase is selected from the group consisting of Glu-tRNA synthetase and Asp-tRNA synthetase.

5. The method according to any of the preceding claims, wherein the expression vector is selected from the group consisting of a viral or non-viral plasmid, cosmid, phagemid, shuttle vector and yak.

6. The method according to claim 5 wherein, the expression vector is an adenovirus vector.

7. The method according to any of the claims 5-6, wherein the vector comprises a tetracycline-dependent regulation system for the expression of the gene.

8. The method according to any of the claims 5-7, wherein the vector comprises a selection marker.

9. The method according to claim 8, wherein the selection marker is hygromicine.

10. The method according to any of the preceding claims, wherein the naturally occurring pathogenic non-discriminating tRNA synthetase comes from a bacterium and the substance is being tested to determine whether it is an antibacterial agent that acts by selectively inhibiting the function of the non-discriminating t-RNA synthetase of bacterial origin expressed into the recombinant mammalian cell.

11. The method according to any of the claims 1-9, wherein the naturally occurring pathogenic non-discriminating tRNA synthetase comes from a fungus and the substance is being tested to determine whether it is an antifungal agent that acts by selectively inhibiting the function of the non-discriminating t-RNA synthetase of fungal origin expressed into the recombinant mammalian cell.

12. The method according to any of the claims 1-9, wherein the naturally occurring pathogenic non-discriminating tRNA synthetase comes from a protozoan and the substance is being tested to determine whether it is an anti-parasite agent that acts by selectively inhibiting the function of the non-discriminating t-RNA synthetase of protozoan origin expressed into the recombinant mammalian cell.

13. The method according to any of the claim 1-9, wherein the naturally occurring pathogenic non-discriminating tRNA synthetase comes from a metazoan and the substance is being tested to determine whether it is an inhibitory agent that acts by selectively inhibiting the function of the non-discriminating t-RNA synthetase of metazoan origin expressed into the recombinant mammalian cell.

14. The method according to any of the preceding claim, wherein the recombinant mammalian cell is a recombinant human cell.

**Patentansprüche**

1. Screening-Verfahren zur Identifizierung eines Wirkstoffkandidaten, der ein Hemmstoff der krankheitserregenden Aminoacyl-tRNA-Synthetase ist, wobei dieses Verfahren die folgenden Verfahrensschritte umfasst:

   a) Erhalten eines Expressionsvektors, der eine Gensequenz umfasst, die für eine natürlich vorkommende krankheitserregende nicht-diskriminierende tRNA-Synthetase kodiert;
   b) Transformation isolierter Säugetierzellen mit dem Expressionsvektor;
   c) Kultivieren der aus (b) erhaltenen rekombinanten Zellen in einem Nährmedium unter Bedingungen, die die Expression der krankheitserregenden tRNA-Synthetase ermöglichen, was zum Zelltod führt;
   d) Bereitstellen einer zu prüfenden Substanz; und
   e) Analyse des resultierenden Zellwachstums, wobei, wenn im Vergleich zum Zellwachstum derselben Kultur in Abwesenheit der zu prüfenden Substanz ein Zellwachstumsanstieg gegeben ist, folglich die Substanz die Aktivität der krankheitserregenden tRNA-Synthetase selektiv hemmt und die normale Zellfunktion nicht beeinträchtigt, was darauf hinweist, dass die besagte Substanz ein Wirkstoffkandidat ist.

2. Verfahren nach Anspruch 1, bei dem der in Verfahrensschritt (a) erhaltene Expressionsvektor auch eine Gensequenz enthält, die für ein tRNA-Substrat der natürlich vorkommenden krankheitserregenden nicht-diskriminierenden tRNA-Synthetase kodiert.

3. Verfahren nach Anspruch 1, bei dem die Säugetierzellen in Verfahrensschritt (b) mithilfe eines zweiten Expressionsvektors transformiert werden, der eine Gensequenz enthält, die für ein tRNA-Substrat der natürlich vorkommenden krankheitserregenden nicht-diskriminierenden tRNA-Synthetase kodiert.

4. Verfahren nach einem der vorstehenden Ansprüche, bei dem die natürlich vorkommende krankheitserregende nicht-diskriminierende tRNA-Synthetase aus der Gruppe ausgewählt wird, die aus Glu-tRNA-Synthetase und Asp-tRNA-Synthetase besteht.

5. Verfahren nach einem der vorstehenden Ansprüche, bei dem der Expressionsvektor aus der Gruppe ausgewählt wird, die aus einem viralen oder nichtviralen Plasmid, Cosmid, Phagemid, Shuttle-Vektor und YAC besteht.

6. Verfahren nach Anspruch 5, bei dem der Expressionsvektor ein adenoviraler Vektor ist.

7. Verfahren nach einem der Ansprüche 5-6, bei dem der Vektor ein Tetracyclinabhängiges Regelsystem für die Expression des Genes umfasst.

8. Verfahren nach einem der Ansprüche 5-7, bei dem der Vektor einen Selektionsmarker umfasst.

9. Verfahren nach Anspruch 8, bei dem der Selektionsmarker Hygromycin ist.

10. Verfahren nach einem der vorstehenden Ansprüche, bei dem die natürlich vorkommende krankheitserregende nicht-diskriminierende tRNA-Synthetase von einem Bakterium stammt, und die Substanz auf ihre Eigenschaft als antibakterieller Wirkstoff hin geprüft wird, der durch selektive Funktionshemmung der nicht-diskriminierenden tRNA-Synthetase bakteriellen Ursprungs, die in die rekombinante Säugetierzelle exprimiert wurde, wirkt.

11. Verfahren nach einem der Ansprüche 1-9, bei dem die natürlich vorkommende krankheitserregende nicht-diskriminierende tRNA-Synthetase von einem Pilz stammt, und die Substanz auf ihre Eigenschaft als antimykotischer Wirkstoff hin geprüft wird, der durch selektive Funktionshemmung der nicht-diskriminierenden tRNA-Synthetase mykotischen Ursprungs, die in die rekombinante Säugetierzelle exprimiert wurde, wirkt.

12. Verfahren nach einem der Ansprüche 1-9, bei dem die natürlich vorkommende krankheitserregende nicht-diskriminierende tRNA-Synthetase von einem Einzeller stammt, und die Substanz auf ihre Eigenschaft als antiparasitärer Wirkstoff hin geprüft wird, der durch selektive Funktionshemmung der nicht-diskriminierenden tRNA-Synthetase protozoischen Ursprungs, die in die rekombinante Säugetierzelle exprimiert wurde, wirkt.

13. Verfahren nach einem der Ansprüche 1-9, bei dem die natürlich vorkommende krankheitserregende nicht-diskriminierende tRNA-Synthetase von einem Vielzeller stammt, und die Substanz auf ihre Eigenschaft als Hemmstoff hin geprüft wird, der durch selektive Funktionshemmung der nicht-diskriminierenden tRNA-Synthetase metazoischen Ursprungs, die in die rekombinante Säugetierzelle exprimiert wurde, wirkt.

14. Verfahren nach einem der vorstehenden Ansprüche, bei dem die rekombinante Säugetierzelle eine rekombinante menschliche Zelle ist.

**Revendications**

1. Procédé de criblage pour identifier un candidat à un médicament qui est un inhibiteur d'aminoacyl-ARNt synthétase pathogène où ledit procédé comprend les étapes suivantes :

   a) l'obtention d'un vecteur d'expression qui comprend une séquence de gène codant pour une ARNt synthétase non discriminatoire pathogène présente naturellement ;
   b) la transformation des cellules de mammifère isolées avec le vecteur d'expression ;
   c) la mise en croissance des cellules recombinantes résultant de (b) dans un milieu de nutriment dans des conditions qui permettent l'expression de l'ARNt synthétase pathogène, ce qui entraîne une mort cellulaire ;
   d) la fourniture d'une substance destinée à être testée ; et
   e) l'analyse de la croissance cellulaire résultante, dans lequel s'il y a une augmentation de croissance cellulaire par rapport à la croissance cellulaire de la même culture en l'absence de la substance destinée à être testée, alors la substance inhibe de façon sélective l'activité de l'ARNt synthétase pathogène et n'affecte pas son orthologue cellulaire, indiquant que ladite substance est un candidat à un médicament.

**2.** Procédé selon la revendication 1, dans lequel le vecteur d'expression obtenu dans l'étape (a) comprend également une séquence de gène codant pour un substrat ARNt de l'ARNt synthétase non discriminatoire pathogène présente naturellement.

**3.** Procédé selon la revendication 1, dans lequel les cellules de mammifère sont transformées dans l'étape (b) en utilisant un deuxième vecteur d'expression comprenant une séquence de gène codant pour un substrat ARNt de l'ARNt synthétase non discriminatoire pathogène présente naturellement.

**4.** Procédé selon l'une quelconque des revendications précédentes, dans lequel l'ARNt synthétase non discriminatoire pathogène présente naturellement est choisie dans le groupe constitué de Glu-ARNt synthétase et d'Asp-ARNt synthétase.

**5.** Procédé selon l'une quelconque des revendications précédentes, dans lequel le vecteur d'expression est choisi dans le groupe constitué d'un plasmide, un cosmide, un phagemide, un vecteur navette et un YAC, viral ou non viral.

**6.** Procédé selon la revendication 5, dans lequel le vecteur d'expression est un vecteur adénovirus.

**7.** Procédé selon l'une quelconque des revendications 5 à 6, dans lequel le vecteur comprend un système de régulation dépendant de la tétracycline pour l'expression du gène.

**8.** Procédé selon l'une quelconque des revendications 5 à 7, dans lequel le vecteur comprend un marqueur de sélection.

**9.** Procédé selon la revendication 8, dans lequel le marqueur de sélection est l'hygromicine.

**10.** Procédé selon l'une quelconque des revendications précédentes, dans lequel l'ARNt synthétase non discriminatoire pathogène présente naturellement provient d'une bactérie et la substance est testée pour déterminer s'il s'agit d'un agent antibactérien qui agit en inhibant de façon sélective la fonction de l'ARNt synthétase non discriminatoire d'origine bactérienne exprimée dans la cellule de mammifère recombinante.

**11.** Procédé selon l'une quelconque des revendications 1 à 9, dans lequel l'ARNt synthétase non discriminatoire pathogène présente naturellement provient d'un champignon et la substance est testée pour déterminer s'il s'agit d'un agent antifongique qui agit en inhibant de façon sélective la fonction de l'ARNt synthétase non discriminatoire d'origine fongique exprimée dans la cellule de mammifère recombinante.

**12.** Procédé selon l'une quelconque des revendications 1 à 9, dans lequel l'ARNt synthétase non discriminatoire pathogène présente naturellement provient d'un protozoaire et la substance est testée pour déterminer s'il s'agit d'un agent antiparasite qui agit en inhibant de façon sélective la fonction de l'ARNt synthétase non discriminatoire d'origine protozoaire exprimée dans la cellule de mammifère recombinante.

**13.** Procédé selon l'une quelconque des revendications 1 à 9, dans lequel l'ARNt synthétase non discriminatoire pathogène présente naturellement provient d'un métazoaire et la substance est testée pour déterminer s'il s'agit d'un agent inhibiteur qui agit en inhibant de façon sélective la fonction de l'ARNt synthétase non discriminatoire d'origine métazoaire exprimée dans la cellule de mammifère recombinante.

**14.** Procédé selon l'une quelconque des revendications précédentes, dans lequel la cellule de mammifère recombinante est une cellule humaine recombinante.

**FIG. 1**

IB: α-GFP

EP 2 041 306 B1

# FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6475726 B
- WO 0204611 A
- EP 785258 A
- EP 1300468 A
- EP 06116233 A

### Non-patent literature cited in the description

- **Weygand-Durasevic I. et al.** Yeast seryl-tRNA synthetase expressed in Escherichia coli recognizes bacterial serine-specific tRNAs in vivo. *Eur. J. Biochem.,* 1993, vol. 214, 869-877
- **Rich, A.** RNA structure and the roots of protein synthesis. *Cold Spring Harb. Symp. Quant. Biol.,* 2001, vol. 66, 1-16
- **Brown, J. R. et al.** Gene descent, duplication, and horizontal transfer in the evolution of glutamyl- and glutaminyl-tRNA synthetases. *J. Mol. Evol.,* 1998, vol. 49, 485-495
- **Hong, K. W.** Retracing the evolution of amino acid specificity in glutaminyl-tRNA synthetase. *FEBS Lett.,* 1999, vol. 434, 149-154
- **Wilcox, M. ; Nirenberg.** Transfer RNA as a cofactor coupling amino acid synthesis with that of protein. *Proc. Natl. Acad. Sci. USA,* 1968, vol. 61, 229-236
- Molecular Cloning: A Laboratory Manual. Cold Spring Harbor Laboratory Press, 2000
- **Rai et al.** Expression systems for production of heterologous proteins. *Current Science,* 2001, vol. 80, 1121-11
- **Edholm, D. et al.** Adenovirus vector designed for expression of toxic proteins. *J. Virology,* 2001, vol. 75, 9579-9584
- **Sauer, B et al.** Cre/lox: one more step in the taming of the genome. *Endocrine,* 2002, vol. 19, 221-228


SEQUENCE LISTING

<110> INSTITUCIÓ CATALANA DE RECERCA I ESTUDIS AVANÇATS FUNDACIÓ PRIVADA PARC CIENTÍFIC DE BARCELONA FUNDACIÓ PRIVADA INSTITUT DE RECERCA BIOMÈDICA

<120> A screening method for identifying new drugs

<130> P669EP00

<140> EP06116233
<141> 2006-06-28

<160> 6

<170> PatentIn version 3.3

<210> 1
<211> 37
<212> DNA
<213> Artificial

<220>
<223> PCR Primer

<400> 1
gtcaccacca tgcttcgttt tgcgccttcg cctacag          37

<210> 2
<211> 44
<212> DNA
<213> Artificial

<220>
<223> PCR Primer

<400> 2
gactcaatgg tgatggtgat gatgtgcttt gagccttaaa actt          44

<210> 3
<211> 30
<212> DNA
<213> Artificial

<220>
<223> PCR Primer

<400> 3
atctccacca tggtgagcaa gggcgaggag          30

<210> 4
<211> 48

<212> DNA
<213> Artificial

<220>
<223> PCR Primer

<400> 4
ctgtaggcga aggcgcaaaa cgaagcttgt acagctcgtc catgccga        48

<210> 5
<211> 48
<212> DNA
<213> Artificial

<220>
<223> sequence to join the amplified green fluorescent protein to the non-canonical Glutamyl t-RNA synthetase

<400> 5
tcggcatgga cgagctgtac aagcttcgtt ttgcgccttc gcctacag        48

<210> 6
<211> 46
<212> DNA
<213> Artificial

<220>
<223> sequence to join the amplified green fluorescent protein to the non-canonical Glutamyl t-RNA synthetase

<400> 6
gatattcaat ggtgatggtg atgatgtgct ttgagcctta aaactt        46